(19) Europäisches Patentamt
European Patent Office
Office européen des brevets

(11) Publication number: **0 578 246 A1**

(12) **EUROPEAN PATENT APPLICATION**

(21) Application number: **93110952.4**

(22) Date of filing: **08.07.93**

(51) Int. Cl.[5]: **C07D 275/03**, A01N 43/80, C07F 7/08, C07D 417/12

(30) Priority: **10.07.92 JP 207304/92**

(43) Date of publication of application: **12.01.94 Bulletin 94/02**

(84) Designated Contracting States: **CH DE ES FR GB IT LI NL**

(71) Applicant: **NIHON NOHYAKU CO., LTD.**
**1-2-5, Nihonbashi**
**Chuo-ku Tokyo(JP)**

(72) Inventor: **Ikeda, Kenichi**
**13-11, Tadain-1-chome**
**Kawanishi-shi(JP)**
Inventor: **Abe, Noboru**
**Maruhashiryo, 4-6, Maruhashicho**
**Nishinomiya-shi(JP)**
Inventor: **Katoh, Chiaki**
**3-29-201, Nankadai-3-chome**
**Kawachinagano-shi(JP)**
Inventor: **Kanaoka, Atsushi**
**3-24-110, Nankadai-3-chome**
**Kawachinagano-shi(JP)**

(74) Representative: **Patentanwälte Grünecker, Kinkeldey, Stockmair & Partner**
**Maximilianstrasse 58**
**D-80538 München (DE)**

(54) **Isothiazole derivatives and processes for preparing the same as well as termite controlling agents comprising the same as active ingredient.**

(57) Isothiazole derivatives represented by general formula (I):

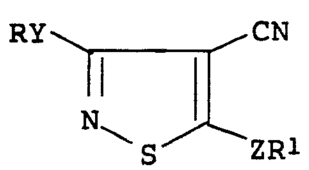

(I)

wherein R, R[1], Y and Z have the same significances as described in the specification, and processes for preparing the same as well as termite controlling agents comprising the same as active ingredient, are disclosed.

EP 0 578 246 A1

BACKGROUND OF THE INVENTION

Field of the Invention

The present invention relates to isothiazole derivatives represented by general formula (I):

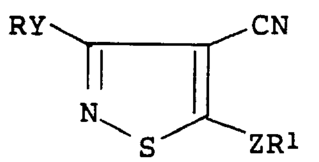

(I)

wherein either R or $R^1$ represents a fluoroalkyl group and the other represents an alkyl group having 1 to 6 carbon atoms; an alkyl group substituted with a member selected from a halogen atom, an alkoxy group having 1 to 6 carbon atoms, an alkoxycarbonyl group having 2 to 7 carbon atoms, an alkylcarbonyl group having 2 to 7 carbon atoms, benzoyl group, an alkylthio group having 1 to 6 carbon atoms, a trialkylsilyl group, a dialkylcarbamoyl group, hydroxy group, cyano group, phenylsulfonyl group, a dialkylamino group, morpholino group, an aryl group, phenoxy group and an aromatic heterocyclic group; a cycloalkyl group; an alkenyl group having 2 to 6 carbon atoms; a haloalkenyl group; an alkynyl group having 2 to 6 carbon atoms; an alkylcarbonyl group having 2 to 7 carbon atoms; benzoyl group; dialkylmethylideneimino group; a dialkylthiocarbamoyl group; a dialkylcarbamoyl group; an alkoxythiocarbonyl group having 2 to 7 carbon atoms; an aryl group or a heterocyclic group; Y represents -O- or -S-; and Z represents -O-, -S-, -N($R^2$) (wherein $R^2$ represents hydrogen atom, an alkyl group having 1 to 6 carbon atoms or an alkynyl group having 2 to 6 carbon atoms, or $R^1$ and $R^2$ may be combined together to form an alkenylene group having 2 to 6 carbon atoms which may be intervened by oxygen atom) or a single bond; and processes for preparing the same as well as termite controlling agents comprising the same as active ingredients.

Related Art

Termites are directly destructive to architecture, living trees, etc. and in addition to woods, also harmful to concrete or polyvinyl products. It is therefore necessary to exterminate or control termites. Serious damages are found in, for example, the foundation, floor or girder of a house, an outdoor wooden pile, an underground polyvinyl-coated wire, etc. so that extermination and control of termites with insecticides are required.

Chemicals of organic chlorine type (e.g., Chlordane, general name) have been frequently used heretofore. However, these insecticides involve problems of persistency, toxicity, irritation, environmental pollutions, etc. and their use is prohibited due to these problems. As substitutes for these insecticides, organic phosphorous insecticides such as chlorpyrifos, phoxim, pyridafenthion and the like, have been used but these organic phosphorous insecticides are often applied to places, e.g., under the floor which are poorly ventilated; an operator absorbs insecticides sprayed in such a small space and might cause toxication by organic phosphorous insecticides. This comes into a problem in industrial hygiene. An additional problem is seen in the duration of these organic phosphorous insecticides. The duration of the effect of these insecticides is shorter than Chlordane conventionally used and hence, there is a concern with their effect of duration as a termite controller required over long periods of time.

SUMMARY OF THE INVENTION

In the state of the art described above, it is desired to develop a novel composition for controlling termites which has a low toxicity to man and beast, has high safety and is readily biodegradable in the soil.

The present inventors have made investigations on isothiazole derivatives. As a result, it has been found that the compounds shown by general formula (I) described above have an excellent effect of controlling termites. The present invention has thus been attained.

The compounds represented by general formula (I) are novel compounds which are not found in any publication. In terms of isothiazole derivatives, however, there are mentioned compounds having a phosphate ester group at the 3-position thereof in West German Patent No. 1,814,249 as having an insecticidal activity, and 3,5-dialkylthio-4-cyanoisothiazoles in Dutch Patent No. 6,703,832 as a pasteurizing

activity. However, there is no report on isothiazole derivatives as exhibiting an activity of controlling termites.

Detailed Description of Preferred Embodiments

In the definitions of R and $R^1$ in the general formula (I) described above, the alkyl group is used to mean a straight or branched chain alkyl group such as methyl, ethyl, isopropyl, n-propyl, n-butyl, isobutyl, sec-butyl, t-butyl, n-pentyl, n-hexyl group, etc.; the fluoroalkyl group is used to mean fluoromethyl, difluoromethyl, trifluoromethyl, 1,1,1-trifluoroethyl, 1,1,2,2-tetrafluoroethyl, 1,1,1,2,2-pentafluoroethyl, 1,1,2,2,3,3,4,4-octafluoropentyl, etc.; the haloalkyl group is used to mean chloromethyl, 1-chloroethyl, 1-chloropropyl, 1-bromopropyl or a fluoroalkyl; the alkenyl group having 2 to 6 carbon atoms is used to mean vinyl, 1-propenyl, 2-propenyl, 1-methyl-2-propenyl, 1,1-dimethyl-2-propenyl, butenyl, pentenyl, hexenyl, etc.; the alkynyl group having 2 to 6 carbon atoms is used to mean 2-propynyl, 1-methyl-2-propynyl, 1,1-dimethyl-2-propynyl, butynyl, pentynyl, hexynyl, etc.; the cycloalkyl group is used to mean cyclopropyl, cyclopentyl, cyclohexyl, etc;, the heterocyclic group is used to mean pyridyl, 3-carboxypyridyl, pyrimidyl, benzothiazolyl, benzimidazolyl, 5-chlorobenzoxazolyl, 1,2,3-triazol-4-yl, 1,2,4-triazol-1-yl, thiazolyl, 2-thiazolin-2-yl, etc.; the alkoxycarbonylalkyl group is used to mean an alkyl group substituted with methoxycarbonyl, ethoxycarbonyl, n-propoxycarbonyl, isopropoxycarbonyl, etc.; the alkylcarbonylalkyl group having 2 to 7 carbon atoms is used to mean an alkyl group substituted with acetyl, propionyl, 2-methylpropionyl, etc.; the arylalkyl group is used to mean an alkyl group substituted with phenyl, alkylphenyl (ex., p-methylphenyl, p-i-propylphenyl), trifluoromethylphenyl, phenoxyphenyl, halophenyl (ex., p-chlorophenyl, pentafluorophenyl), biphenylmethyl, etc.; the alkyl group substituted with an aromatic heterocyclic group is used to mean an alkyl group substituted with 3-pyridyl, 2-pyridyl, 1,2,4-triazol-1-yl, etc.; the aryl group is used to mean a phenyl group, a phenyl group substituted with 1 to 3 groups selected from an alkyl group having 1 to 6 carbon atoms, fluorine, chlorine, bromine, nitro, hydroxy, carboxyl, an alkoxycarbonyl, an alkoxycarbonylalkoxy, an alkoxy, phenoxy, trifluoromethyl and the like; the alkyl-substituted carbamoyl group is used to mean a carbamoyl group substituted with 1 or 2 straight or branched chain alkyl groups such as methyl, ethyl, n-propyl, isopropyl, n-butyl, t-butyl, n-pentyl, n-hexyl, etc.; the alkyl-substituted thiocarbamoyl group is used to mean a thiocarbamoyl group substituted with 1 or 2 straight or branched chain alkyl groups such as methyl, ethyl, n-propyl, isopropyl, n-butyl, t-butyl, n-pentyl, n-hexyl, etc. Furthermore, the term "alkyl" means in the present specification an alkyl group having one to six carbon atoms unless otherwise specified.

Among the compounds of the present invention, those which are particularly useful as a termite controlling agent will be shown below:

3-difluoromethylthio-4-cyano-5-n-propylthioisothiazole, 3-difluoromethylthio-4-cyano-5-i-propylthioisothiazole, 3-difluoromethylthio-4-cyano-5-s-butylthioisothiazole, 3-difluoromethylthio-4-cyano-5-cyclopentylthioisothiazole, 3-difluoromethylthio-4-cyano-5-cyclohexylthioisothiazole, 3-difluoromethylthio-4-cyano-5-isopropoxyethylthioisothiazole, 3-difluoromethylthio-4-cyano-5-(2-methylallyl)thioisothiazole, 3-difluoromethylthio-4-cyano-5-propargylthioisothiazole, 3-difluoromethoxy-4-cyano-5-isopropylthioisothiazole, 3-difluoromethoxy-4-cyano-5-s-butylthioisothiazole, 3-difluoromethoxy-4-cyano-5-t-butylthioisothiazole, 3-difluoromethoxy-4-cyano-5-cyclopentylthioisothiazole, 3-difluoromethoxy-4-cyano-5-cyclohexylthioisothiazole, 3-difluoromethoxy-4-cyano-5-s-penthylthioisothiazole, 3-difluoromethoxy-4-cyano-5-methallylthioisothiazole, 3-difluoromethoxy-4-cyano-5-propargylthioisothiazole, 3-difluoromethoxy-4-cyano-5-ethoxyethylthioisothiazole, 3-difluoromethoxy-4-cyano-5-ethoxyisothiazole, 3-difluoromethoxy-4-cyano-5-i-propoxyisothiazole, 3-difluoromethoxy-4-cyano-5-i-butoxyisothiazole, 3-difluoromethoxy-4-cyano-5-ethoxyethoxyisothiazole, 3-difluoromethoxy-4-cyano-5-phenoxyisothiazole, 3-difluoromethoxy-4-cyano-5-m-methylphenoxyisothiazole, 3-difluoromethoxy-4-cyano-5-p-fluorophenylisothiazole, 3-difluoromethoxy-4-cyano-5-propargyloxyisothiazole, 3-i-propoxy-4-cyano-5-difluoromethylthioisothiazole, 3-s-butoxy-4-cyano-5-difluoromethylthioisothiazole, 3-s-pentyloxy-4-cyano-5-difluoromethylthioisothiazole, 3-cyclopentyloxy-4-cyano-5-difluoromethylthioisothiazole, 3-cyclohexyloxy-4-cyano-5-difluoromethylthioisothiazole, 3-trimethylsilylmethoxy-4-cyano-5-difluoromethylthioisothiazole, 3-ethoxyethoxy-4-cyano-5-difluoromethylthioisothiazole, 3-propargyloxy-4-cyano-5-difluoromethylthioisothiazole, and 3-allyloxy-4-cyano-5-difluoromethylthioisothiazole.

The compounds represented by general formula (I) can be synthesized according to Processes A, B, C and D.

Process A:

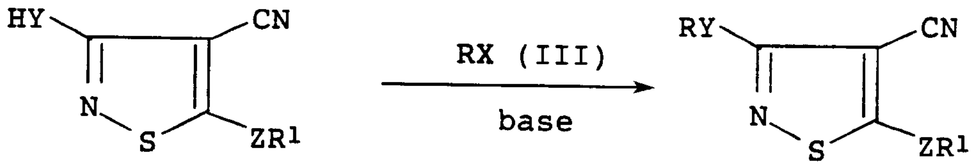


(II) (Ib)

wherein R, $R^1$, Y and Z have the same significances as described above, and X represents a halogen atom.

That is, the isothiazole derivatives represented by general formula (I) are obtained by reacting compounds shown by general formula (II) with compounds shown by general formula (III) in the presence of a base.

Process B:

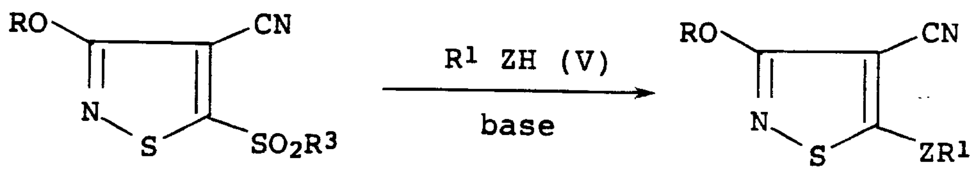


(IV) (Ia)

wherein R, $R^1$ and Z have the same significances as described above, and $R^3$ represents an alkyl group having 1 to 6 carbon atoms.

That is, the isothiazole derivatives represented by general formula (Ia) are obtained by reacting compounds shown by general formula (IV) with nucleophilic compounds shown by general formula (V) in the presence of a base.

Process C:

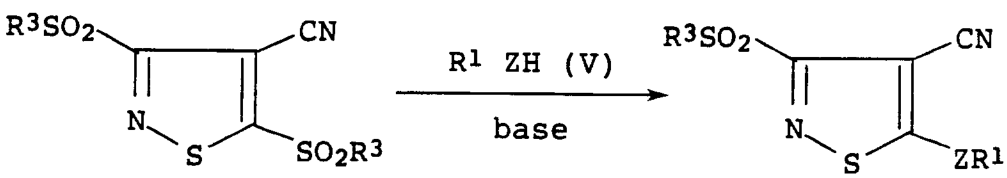


(VI) (VII)

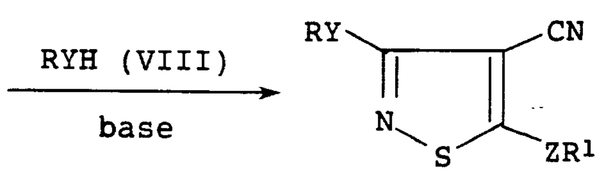


(Ib)

wherein R, $R^1$, $R^3$, Y and Z have the same significances as described above.

That is, the isothiazole derivatives represented by general formula (Ib) are obtained by reacting compounds shown by general formula (VI) with compounds shown by general formula (V) in the presence of a base to synthesize compounds shown by general formula (VII) followed by reacting with nucleophilic compounds shown by general formula (VIII) after or without isolation of the compounds (VII).

Process D:

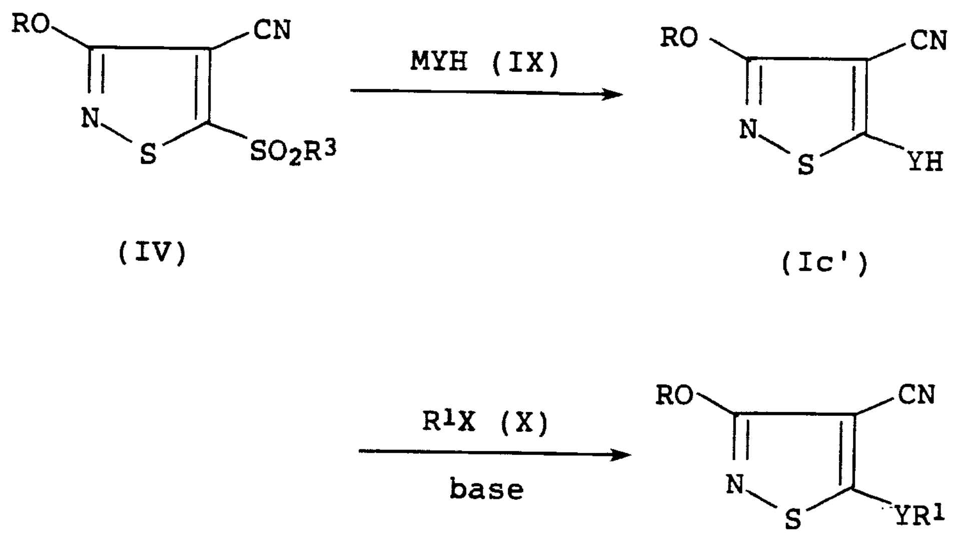


(IV) (Ic')

(Ic)

wherein R, $R^1$, $R^3$, X and Y have the same significances as described above.

That is, the isothiazole derivatives represented by general formula (Ic) are obtained by reacting compounds shown by general formula (IV) with bases shown by general formula (IX) followed by reacting with compounds shown by general formula (X).

The compounds shown by general formula (IV) used in the reaction described above are obtained by oxidizing the corresponding alkylthio compounds with oxon in an inert solvent.

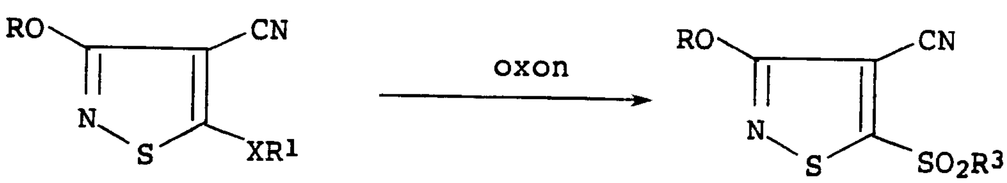


(II) (IV)

wherein R, $R^1$, $R^3$ and Z have the same significances as described above.

In a similar manner, the compounds represented by general formulae (VI) are obtained.

The compounds represented by general formula (II) were synthesized in a conventional manner [J.O.C., 28, 2163 (1962), J.O.C., 29, 665 (1964)).

In Processes A, B, C and D, any solvents are usable so long as they are inert to the reactions. Specific examples of such solvents that can be used include alcohols such as methanol, ethanol, isopropanol, t-butanol, diethylene glycol, etc.; ketones such as acetone, methyl ethyl ketone, cyclohexanone, etc.; ethers such as diethyl ether, diisopropyl ether, dimethoxyethane, tetrahydrofuran, dioxane, monoglyme, diglyme, etc.; halogenated hydrocarbons such as dichloroethane, chloroform, carbon tetrachloride, tetrachloroethane, etc.; aromatic hydrocarbons such as benzene, chlorobenzene, nitrobenzene, toluene, etc.; nitriles such as acetonitrile, etc.; dimethylformamide, dimethylsulfoxide, water, and a solvent mixture of these solvents in combination. In the case that a double phase reaction is carried out using a solvent mixture, there may be

used an interphase transfer catalyst such as triethylbenzyl ammonium chloride, trioctylmethyl ammonium chloride, etc.

As the base, an inorganic base or an organic base may be used. Examples of the inorganic base include alkali metal or alkaline earth metal carbonates such as sodium carbonate, potassium carbonate, calcium carbonate, sodium hydrogencarbonate, etc.; alkali metal or alkaline earth metal hydroxides such as sodium hydroxide, potassium hydroxide, calcium hydroxide, etc.; alkali metal hydrides such as lithium hydride, sodium hydride, etc. As the organic bases there may be used diethylamine, triethylamine, sodium methoxide, potassium t-butoxide, pyridine, benzyltrimethyl ammonium hydroxide, etc.

The reaction temperature may be appropriately chosen in the range of from 20 °C to 100 °C, preferably in the range of 40 °C to 60 °C. The reaction time varies depending upon reaction temperature and reaction scale but may be chosen generally in the range of 30 minutes to 12 hours.

The reaction proceeds in an equimolar relation so that respective reactants may be used in equimolar amounts but either may be used in an excess amount. The base may be used in an equimolar amount based on 1 mole of the compounds represented by general formula (II), (V), or (X); of course, the base may also be used in an excess amount.

Specific examples of the compounds represented by general formula (I) are given together with melting point or refractive index of the each compound in Table 1, but the present invention is not deemed to be limited thereto.

Table 1

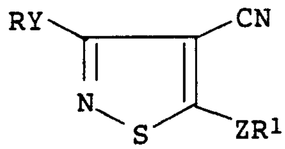

(I)

| No. | R | Y | $R^1$ | Z | Physical Property |
|---|---|---|---|---|---|
| 1 | $F_2CH$- | O | $CH_3$- | S | 55-56°C |
| 2 | " | " | $C_2H_5$- | " | 42-43°C |
| 3 | " | " | n-$C_3H_7$- | " | 27-28°C |
| 4 | " | " | i-$C_3H_7$- | " | 24-25°C |
| 5 | " | " | n-$C_4H_9$- | " | $n_D^{19}$ 1.5170 |
| 6 | " | " | i-$C_4H_9$- | " | $n_D^{18}$ 1.5185 |
| 7 | " | " | s-$C_4H_9$- | " | $n_D^{23}$ 1.5641 |
| 8 | " | " | t-$C_4H_9$- | " | 38-41°C |
| 9 | " | " | 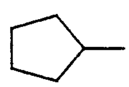 | " | 43-44°C |
| 10 | " | " | 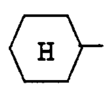 | " | 47-48°C |
| 11 | " | " | s-$C_5H_{11}$- | " | $n_D^{20}$ 1.5183 |
| 12 | " | " | $CH_2$=CH-$CH_2$- | " | $n_D^{20}$ 1.5344 |
| 13 | " | " | $CH_2$=C($CH_3$)$CH_2$- | " | $n_D^{20}$ 1.5264 |

Table 1 (Cont'd)

| No. | R | Y | $R^1$ | Z | Physical Property |
|---|---|---|---|---|---|
| 14 | $F_2CH-$ | O | $CH_3CH=CH-CH_2-$ | S | $n_D^{20}$ 1.5220 |
| 15 | " | " | $CH\equiv C-CH_2-$ | " | 39-41°C |
| 16 | " | " | $CH_3OC_2H_4-$ | " | 53-54°C |
| 17 | " | " | $C_2H_5OC_2H_4-$ | " | 49-50°C |
| 18 | " | " | $i-C_3H_7OC_2H_4-$ | " | $n_D^{20}$ 1.5284 |
| 19 | " | " | 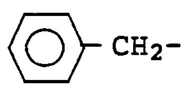 | " | 53-54°C |
| 20 | " | " | 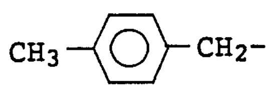 | " | 63-64°C |
| 21 | " | " | 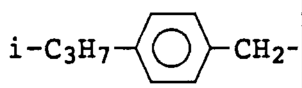 | " | 55-56°C |
| 22 | " | " | 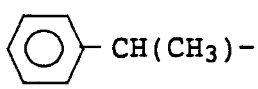 | " | 61-62°C |
| 23 | " | " | 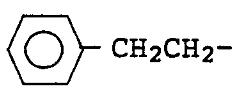 | " | 71-72°C |
| 24 | " | " | $Cl-CH=CH-CH_2-$ | " | 45-46°C |
| 25 | " | " | $Cl_2C=CH-CH_2-$ | " | 34-36°C |

Table 1 (Cont'd)

| No. | R | Y | $R^1$ | Z | Physical Property |
|---|---|---|---|---|---|
| 26 | $F_2CH$- | O | 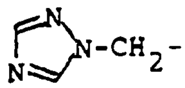 | S | 101-102°C |
| 27 | " | " | 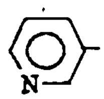 $CH_2$- | " | 125-126°C |
| 28 | " | " | 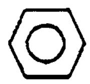 $OCH_2CH_2$- | " | 101-102°C |
| 29 | " | " | 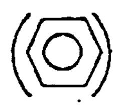 2CH- | " | 123-124°C |
| 30 | " | " | $CH_3O_2C-CH_2$- | " | 69-70°C |
| 31 | " | " | $C_2H_5O_2C-CH(CH_3)$- | " | 54-55°C |
| 32 | " | " | $CH_3C(O)CH_2$- | " | 49-50°C |
| 33 | " | " | 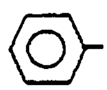 $C(O)CH_2$- | " | 134-135°C |
| 34 | " | " | $NC-CH_2CH_2$- | " | 72-73°C |
| 35 | " | " | $CH_3SCH_2$- | " | 85-86°C |
| 36 | " | " | $(CH_3)_3SiCH_2$- | " | 41-42°C |
| 37 | " | " | $CH_3C(O)$- | " | 122-123°C |

Table 1 (Cont'd)

| No. | R | Y | $R^1$ | Z | Physical Property |
|---|---|---|---|---|---|
| 38 | $F_2CH-$ | O | 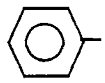 C(O)- | S | 154-155°C |
| 39 | " | " | $(CH_3)_2NC(O)CH_2-$ | " | 121-122°C |
| 40 | " | " | $(C_2H_5)_2NC(S)-$ | " | 91-92°C |
| 41 | " | " | $(C_2H_5)_2NC(O)-$ | " | 64-65°C |
| 42 | " | " | $i-C_3H_7OC(S)-$ | " | 88-89°C |
| 43 | " | " | $HOCH_2CH_2-$ | " | 43-44°C |
| 44 | " | " | 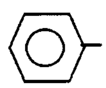 $SO_2CH_2-$ | " | 178-179°C |
| 45 | " | " | $(CH_3)_2NCH_2CH_2-$ | " | 77-78°C |
| 46 | " | " | 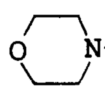 $-CH_2CH_2-$ | " | 64-65°C |
| 47 | " | " | $CF_3CH_2-$ | " | 45-46°C |
| 48 | " | " | $ClCH_2CH_2-$ | " | 62-63°C |
| 49 | " | " | $CF_3CF_2CH_2-$ | " | 38-39°C |
| 50 | " | " | $HCF_2CF_2CH_2-$ | " | 40-41°C |

Table 1 (Cont'd)

| No. | R | Y | $R^1$ | Z | Physical Property |
|---|---|---|---|---|---|
| 51 | $F_2CH-$ | O | $HCF_2(CF_2)_3CH_2$ | S | 29-30°C |
| 52 | " | " | 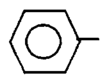 | " | 93-94°C |
| 53 | " | " | 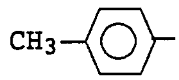 | " | 92-93°C |
| 54 | " | " | 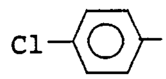 | " | 132-133°C |
| 55 | " | " | 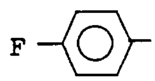 | " | 77-78°C |
| 56 | " | " | 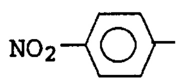 | " | 116-117°C |
| 57 | " | " | 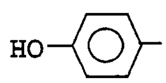 | " | 161-162°C |
| 58 | " | " | 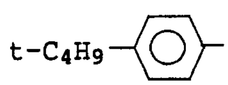 | " | 52-53°C |
| 59 | " | " | 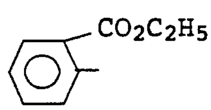 | " | 111-112°C |
| 60 | " | " | 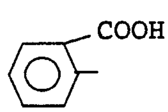 | " | 166-168°C |

Table 1 (Cont'd)

| No. | R | Y | $R^1$ | Z | Physical Property |
|---|---|---|---|---|---|
| 61 | $F_2CH-$ | O | 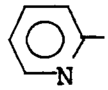 | S | 173-174°C |
| 62 | " | " | 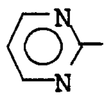 | " | 147°C |
| 63 | " | " | 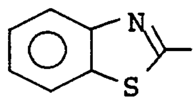 | " | 153-154°C |
| 64 | " | " | 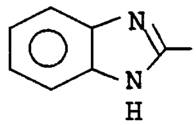 | " | 197-198°C |
| 65 | " | " | 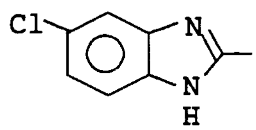 | " | 201-202°C |
| 66 | " | " | 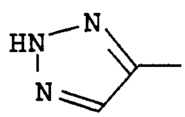 | " | 156°C |
| 67 | " | " | 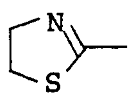 | " | 112-113°C |
| 68 | " | " | 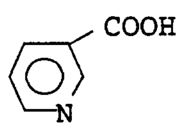 | " | 250°C (dec.) |
| 69 | " | " | 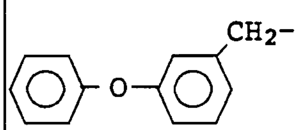 | " | 56-57°C |
| 70 | " | " | 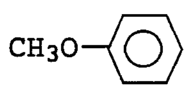 | " | 116-117°C |

Table 1 (Cont'd)

| No. | R | Y | $R^1$ | Z | Physical Property |
|---|---|---|---|---|---|
| 71 | $CF_3CH_2$- | O | $CH_3$- | S | 58-59°C |
| 72 | " | " | $C_2H_5$- | " | 43-44°C |
| 73 | " | " | i-$C_3H_7$- | " | 29-30°C |
| 74 | " | " | 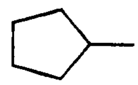 | " | 41-42°C |
| 75 | " | " | 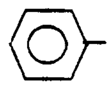 $CH_2$- | " | 61-62°C |
| 76 | " | " | $CH_3C(O)CH_2$- | " | 51-52°C |
| 77 | $HCF_2CF_2CH_2$- | " | $CH_3$- | " | 45-46°C |
| 78 | " | " | i-$C_3H_7$- | " | 39-40°C |
| 79 | " | " | s-$C_4H_9$- | " | $n_D^{19}$ 1.5346 |
| 80 | " | " | 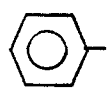 $CH_2$- | " | 78-79°C |
| 81 | $CF_3CF_2CH_2$- | " | $C_2H_5$- | " | 29-30°C |
| 82 | " | " | i-$C_4H_9$- | " | 25-27°C |
| 83 | " | " | 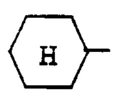 | " | 45-46°C |

Table 1 (Cont'd)

| No. | R | Y | $R^1$ | Z | Physical Property |
|---|---|---|---|---|---|
| 84 | $CF_3CF_2CH_2$- | O | $C_2H_5OC_2H_4$- | S | 41-42°C |
| 85 | " | " | 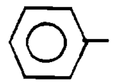 | " | 94-95°C |
| 86 | $CH_3$- | " | $F_2CH$- | " | 29-30°C |
| 87 | " | " | $HCF_2CF_2CH_2$- | " | 38-40°C |
| 88 | $CH{\equiv}C-CH_2$- | " | $F_2CH$- | " | 41-42°C |
| 89 | $C_2H_5$- | " | $CF_3CF_2CH_2$- | " | 61-62°C |
| 90 | $CH_2=CH-CH_2$- | " | $F_2CH$- | " | 43-44°C |
| 91 | 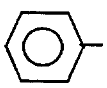 $CH_2$- | " | $F_2CH$- | " | 49-50°C |
| 92 | 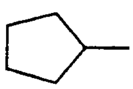 | " | $CF_3CH_2$- | " | 46-47°C |
| 93 | $F_2CH$- | " | $F_2CH$- | " | 43-44°C |
| 94 | $CF_3CH_2$- | " | $CF_3CH_2$- | " | 39-40°C |
| 95 | $CF_3CH_2$- | " | $CF_3CF_2CH_2$- | " | 24-25°C |
| 96 | $HCF_2CF_2CH_2$- | " | $HCF_2CF_2CH_2$- | " | 31 -32°C |

Table 1 (Cont'd)

| No. | R | Y | $R^1$ | Z | Physical Property |
|---|---|---|---|---|---|
| 97 | $F_2CH$- | O | $CH_3$- | S | 59-60°C |
| 98 | " | " | $C_2H_5$- | " | 46-47°C |
| 99 | " | " | n-$C_3H_7$- | " | $n_D^{19}$ 1.5366 |
| 100 | " | " | i-$C_3H_7$- | " | $n_D^{19}$ 1.5470 |
| 101 | " | " | n-$C_4H_9$- | " | $n_D^{19}$ 1.5264 |
| 102 | " | " | s-$C_4H_9$- | " | $n_D^{23}$ 1.5641 |
| 103 | " | " | 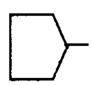 | " | 62-63°C |
| 104 | " | " | 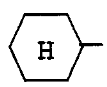 | " | 65-66°C |
| 105 | " | " | 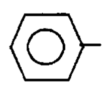 $CH_2$- | " | 44-46°C |
| 106 | " | " | i-$C_3H_7OC_2H_4$- | " | $n_D^{19}$ 1.5341 |
| 107 | " | " | $CH_2$=CH-$CH_2$- | " | 44-45°C |
| 108 | " | " | CH≡C-$CH_2$- | " | 41-42°C |
| 109 | " | " | 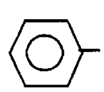 $OCH_2CH_2$- | " | 63-65°C |

Table 1 (Cont'd)

| No. | R | Y | $R^1$ | Z | Physical Property |
|---|---|---|---|---|---|
| 110 | $F_2CH-$ | S | $CF_3CH_2-$ | S | 44-45°C |
| 111 | " | " | 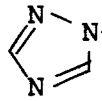 N-$CH_2-$ | " | 51-52°C |
| 112 | " | " | 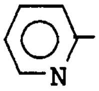 $CH_2-$ | " | 93-94°C |
| 113 | " | " | $(CH_3)_3SiCH_2-$ | " | 71-72°C |
| 114 | " | " | $(CH_3)_3CC(O)CH_2-$ | " | 46-47°C |
| 115 | " | " | $NCCH_2CH_2-$ | " | 55-56°C |
| 116 | $HCF_2CF_2CH_2-$ | " | $CH_3-$ | " | 40-42°C |
| 117 | $CF_3CH_2-$ | " | $CF_3CH_2-$ | " | 39-40°C |
| 118 | $HCF_2CF_2CH_2-$ | " | $HCF_2CF_2CH_2-$ | " | 46-47°C |
| 119 | $CF_3CF_2CH_2-$ | " | $CF_3CF_2CH_2-$ | " | 49-50°C |
| 120 | s-$C_4H_9$ | " | $F_2CH-$ | " | $n_D^{23}$ 1.5641 |
| 121 | $F_2CH-$ | O | $CH_3-$ | O | 36-37°C |
| 122 | " | " | $C_2H_5-$ | " | 65-66°C |
| 123 | " | " | n-$C_3H_7-$ | " | 46-47°C |
| 124 | " | " | i-$C_3H_7-$ | " | 49-50°C |

Table 1 (Cont'd)

| No. | R | Y | $R^1$ | Z | Physical Property |
|---|---|---|---|---|---|
| 125 | $F_2CH-$ | O | $n-C_4H_9-$ | O | $n_D^{20}$ 1.5462 |
| 126 | " | " | $i-C_4H_9-$ | " | $n_D^{20}$ 1.5514 |
| 127 | " | " | $s-C_4H_9-$ | " | $n_D^{20}$ 1.5332 |
| 128 | " | " | $C_2H_5OC_2H_4-$ | " | 48-49°C |
| 129 | " | " | 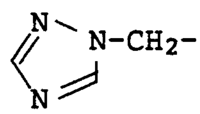 | " | 56-57°C |
| 130 | " | " | 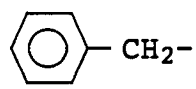 | " | 106-107°C |
| 131 | " | " | 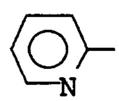 | " | 126-128°C |
| 132 | " | " | 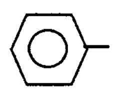 | " | 54-55°C |
| 133 | " | " | 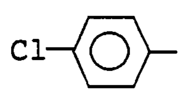 | " | 103-104°C |
| 134 | " | " | 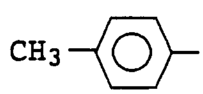 | " | 141-142°C |
| 135 | " | " | 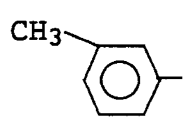 | " | 58-59°C |

Table 1 (Cont'd)

| No. | R | Y | $R^1$ | Z | Physical Property |
|---|---|---|---|---|---|
| 136 | $F_2CH-$ | O | 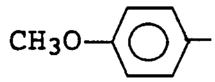 | O | 126-127°C |
| 137 | " | " | 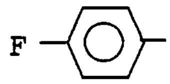 | " | 51-52°C |
| 138 | " | " | 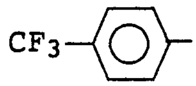 | " | 47-48°C |
| 139 | " | " | 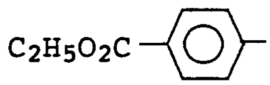 | " | 62-63°C |
| 140 | " | " | 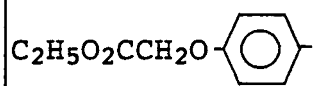 | " | 111-112°C |
| 141 | " | " | 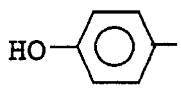 | " | 121-122°C |
| 142 | " | " | 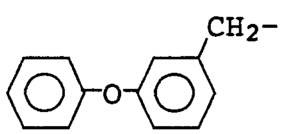 | " | 81-82°C |
| 143 | " | " | 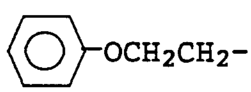 | " | 95-96°C |
| 144 | " | " | $CH{\equiv}C-CH_2-$ | " | 41-42°C |

Table 1 (Cont'd)

| No. | R | Y | $R^1$ | Z | Physical Property |
|---|---|---|---|---|---|
| 145 | $F_2CH-$ | O | $CF_3CH_2-$ | O | 56-57°C |
| 146 | " | " | $CF_3CF_2CH_2-$ | " | 43-44°C |
| 147 | " | " | $HCF_2(CF_2)_3CH_2-$ | " | 44-45°C |
| 148 | " | " | $(CH_3)_2C=N-$ | " | 59-60°C |
| 149 | " | " | 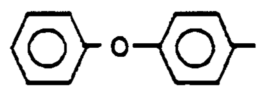 | " | 131-132°C |
| 150 | " | " | 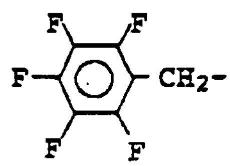 | " | 76-77°C |
| 151 | $CH_3-$ | " | $F_2CH-$ | " | 32-33°C |
| 152 | " | " | $CF_3CH_2-$ | " | 57-58°C |
| 153 | $C_2H_5-$ | " | " | " | 44-45°C |
| 154 | $i-C_3H_7-$ | " | " | " | 103-105°C |
| 155 | $s-C_4H_9-$ | " | " | " | 96-97°C |
| 156 | 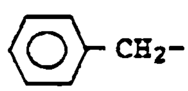 | " | " | " | 130-131°C |
| 157 | 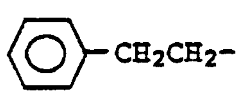 | " | $HCF_2CF_2CH_2-$ | " | 105-106°C |

Table 1 (Cont'd)

| No. | R | Y | $R^1$ | Z | Physical Property |
|---|---|---|---|---|---|
| 158 | $CH_2=CH-CH_2-$ | O | $CF_3CH_2-$ | O | 49-50°C |
| 159 | $CH\equiv C-CH_2-$ | " | " | " | 51-52°C |
| 160 | $C_2H_5OC_2H_4-$ | " | " | " | 62-63°C |
| 161 | $CH_3O_2C-CH_2-$ | " | " | " | 88-90°C |
| 162 | $NC-CH_2-$ | " | " | " | 92-93°C |
| 163 | $(CH_3)_3SiCH_2-$ | " | " | " | 111-113°C |
| 164 | 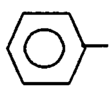 | " | " | " | 161-162°C |
| 165 | 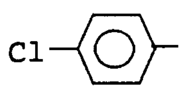 | " | " | " | 186-188°C |
| 166 | $CF_3CH_2-$ | " | " | " | 46-47°C |
| 167 | " | " | 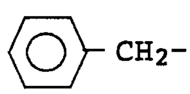 | " | 71-72°C |
| 168 | $HCF_2CF_2CH_2-$ | " | $HCF_2CF_2CH_2-$ | " | 42-44°C |
| 169 | $CF_3CF_2CH_2-$ | " | $CF_3CF_2CH_2-$ | " | 41-43°C |

Table 1 (Cont'd)

| No. | R | Y | $R^1$ | Z | Physical Property |
|---|---|---|---|---|---|
| 170 | $CF_3CF_2CH_2$- | O | 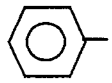 | O | 45-47°C |
| 171 | $HCF_2(CF_2)_3CH_2$- | " | $HCF_2(CF_2)_3CH_2$- | " | 36-37°C |
| 172 | $CH_3$- | " | $HCF_2CF_2CH_2$- | " | 47-48°C |
| 173 | t-$C_4H_9$- | " | $CF_3CH_2$- | " | 44-45°C |
| 174 | 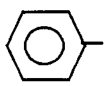 $CH_2$- | " | " | " | 67-68°C |
| 175 | 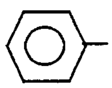 | " | $CF_3CF_2CH_2$- | " | 106-107°C |
| 176 | $F_2CH$- | O | $CH_3$- | NH | 90-91°C |
| 177 | " | " | $C_2H_5$- | " | 70-71°C |
| 178 | " | " | i-$C_3H_7$- | " | 160-161°C |
| 179 | " | " | n-$C_4H_9$- | " | 121-122°C |
| 180 | " | " | s-$C_4H_9$- | " | 113-114°C |
| 181 | " | " | 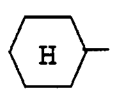 | " | 179-180°C |

Table 1 (Cont'd)

| No. | R | Y | $R^1$ | Z | Physical Property |
|---|---|---|---|---|---|
| 182 | $F_2CH$- | O | 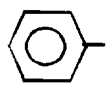 $CH_2$- | NH | 72-73°C |
| 183 | " | " | $CH_3$- | $NCH_3$ | 92-93°C |
| 184 | " | " | $C_2H_5$- | $NC_2H_5$ | 46-47°C |
| 185 | " | " | $-CH_2CH_2CH_2CH_2-$ | N | 105-106°C |
| 186 | " | " | $-(CH_2)_5-$ | " | 117-118°C |
| 187 | " | " | $-CH_2CH_2O-CH_2CH_2-$ | " | 121-122°C |
| 188 | " | " | $CF_3CH_2$- | NH | 61-62°C |
| 189 | " | " | $CF_3$ 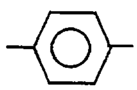 $CH_2$- | " | 121-123°C |
| 190 | " | " | 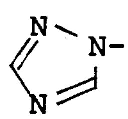 | " | 56-57°C |
| 191 | " | " | 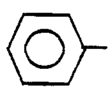 | " | 136-137°C |
| 192 | $CF_3CH_2$- | " | 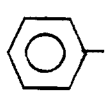 $CH_2$- | " | 51-52°C |
| 193 | $CF_3CF_2CH_2$- | " | $CH_3$- | $NCH_3$ | 96-97°C |

Table 1 (Cont'd)

| No. | R | Y | $R^1$ | Z | Physical Property |
|---|---|---|---|---|---|
| 194 | $CF_3CF_2CH_2$- | O | $-(CH_2)_5-$ | N | 117-118°C |
| 195 | $CH_3$- | S | $CF_3CH_2$- | NH | 101-102°C |
| 196 | 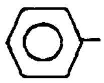 $CH_2$- | " | " | " | 136-137°C |
| 197 | 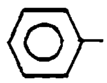 | " | " | " | 166-167°C |
| 198 | i-$C_3H_7$- | " | $F_2CH$- | S | $n_D^{26}$ 1.5221 |
| 199 | i-$C_4H_9$- | " | " | " | $n_D^{26}$ 1.5210 |
| 200 | s-$C_4H_9$- | " | " | " | $n_D^{26}$ 1.5125 |
| 201 | s-$C_5H_{11}$- | " | " | " | $n_D^{26}$ 1.5150 |
| 202 | 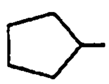 | " | " | " | $n_D^{26}$ 1.5160 |
| 203 | 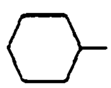 | " | " | " | $n_D^{26}$ 1.5166 |
| 204 | $(CH_3)_3SiCH_2$- | " | " | " | $n_D^{26}$ 1.5218 |
| 205 | $C_2H_5OCH_2CH_2$- | " | " | " | 44-45°C |
| 206 | $HC\equiv CCH_2$- | " | " | " | $n_D^{25}$ 1.5234 |
| 207 | $H_2C=CHCH_2$- | " | " | " | $n_D^{25}$ 1.5238 |

Hereafter the present invention is described with reference to the examples below but is not deemed to be limited thereto.

Example 1

Synthesis of 3-difluoromethoxy-4-cyano-5-methylthioisothiazole (Compound 1)

After 17.2 g (0.1 mol) of 3-hydroxy-4-cyano-5-methylthioisothiazole was dissolved in 100 ml of dioxane, 20 ml of 20% sodium hydroxide aqueous solution was added to the solution. With stirring, difluorochloromethane was blown into the mixture. While dropwise adding 180 ml of 20% sodium hydroxide aqueous solution, difluorochloromethane was further blown into the mixture over 30 minutes so that the reaction solution became about 60 to 70 °C. After cooling, the oily substance separated was extracted with ethyl acetate. After washing with water and drying, the extract was distilled under reduced pressure. The thus obtained crystals were recrystallized from hexane to give 15 g (yield, 67.6%) of white crystals showing a melting point of 55 to 56 °C.

$^1$HNMR ($CDCl_3$) $\sigma$: 2.66 (3H, s, $SCH_3$), 6.98-7.60 (1H, t, $-CHF_2$)

Example 2

Synthesis of 3-difluoromethoxy-4-cyano-5-cyclopentylthioisothiazole (Compound 9)

After 22.8 g (0.1 mol) of 3-hydroxy-4-cyano-5-cyclopentylthioisothiazole was added to 200 ml of toluene, difluorochloromethane was blown into the mixture while dropwise adding 200 ml of 30% potassium hydroxide aqueous solution with stirring. After continuing to blow for 30 minutes, 200 ml of water was added to the mixture and the toluene layer was fractionated. After washing with water and drying, toluene was distilled off under reduced pressure. The thus obtained crude crystals were recrystallized from hexane to give 20 g (yield, 72%) of white crystals showing a melting point of 43 to 44 °C.

$^1$HNMR ($CDCl_3$) $\sigma$: 1.05-1.15 (4H, t), 1.80-2.05 (4H, m), 6.90-7.60 (1H, t, J = 6.6 Hz)

Example 3

Synthesis of 2,2,2-trifluoroethoxy-4-cyano-5-ethylthioisothiazole (Compound 72)

After 1.86 g (0.01 mol) of 3-hydroxy-4-cyano-5-ethylthioisothiazole was dissolved in 20 ml of dimethylformamide, 0.7 g of potassium carbonate was added to the solution. With stirring, 2.2 g of trifluoroethyl iodide ($CH_3CH_2I$) was further added to the mixture. The reaction solution was heated to 40 to 50 °C followed by stirring for 3 hours. After cooling, water was added. The mixture was then extracted with 50 ml of ethyl acetate. After washing with water and drying, the extract was concentrated. To the thus obtained oily substance was added 10 ml of hexane. The mixture was heated to dissolve. Insoluble matters were filtered off and the filtrate was kept in a refrigerator at 3 °C. The resulting crystals were collected by filtration to give 0.4 g (yield, 14.9%) of white crystals showing a melting point of 43 to 44 °C.

$^1$HNMR ($CDCl_3$) $\sigma$: 1.40-1.52 (3H, t, $-SCH_2CH_3$), 3.05-3.20 (2H, q, $-SCH_2CH_3$), 4.70-4.82 (2H, q, $-CH_2CF_3$)

Example 4

Synthesis of 3-difluoromethoxy-4-cyano-5-trimethylsilylmethylthioisothiazole (Compound 36)

After 2.4 g (0.01 mol) of 3-hydroxy-4-cyano-5-trimethylsilylmethylthioisothiazole was dissolved in 50 ml of dimethylformamide, 0.7 g of potassium carbonate was added to the solution. With stirring, the mixture was heated to 50 to 70 °C while blowing difluorochloromethane into the mixture. After blowing for an hour with stirring, water was added. The mixture was extracted with 100 ml of ethyl acetate. After washing with water and drying, the extract was concentrated under reduced pressure. The resulting residue was dissolved in hexane and insoluble matters were removed. The filtrate was kept in a refrigerator at 3 °C.
The resulting crystals were collected by filtration and dried to give 0.3 g (yield, 10.2%) of white crystals showing a melting point of 41 to 42 °C.

Example 5

Synthesis of 3-(2,2,3,3-tetrafluoropropyl)thiomethoxy-4-cyano-5-methylthioisothiazole (Compound 116)

After 2.1 g (0.01 mol) of 3-mercapto-4-cyano-5-methylthio-isothiazole sodium salt was dissolved in 20 ml of dimethylformamide, 2.5 g of tetrafluoropropyl iodide ($HCF_2CF_2CH_2I$) was added to the solution. The reaction solution was heated to 50 to 70 ° C followed by stirring. After cooling, water was added. The mixture was extracted with 100 ml of ethyl acetate. After washing with water and drying, the extract was concentrated under reduced pressure to give about 2 g of crude crystals. The crystals were dissolved in hexane and insoluble matters were removed. The filtrate was kept in a refrigerator at 3 ° C. The resulting crystals were collected by filtration and dried to give 0.5 g (yield, 16.0%) of crystals showing a melting point of 40 to 42 ° C.

Example 6

Synthesis of 3-trifluoroethylthio-4-cyano-5-trifluoroethylthioisothiazole (Compound 117)

After 2.2 g (0.01 mol) of 3,5-dimercapto-4-cyanoisothiazole sodium salt was dissolved in 20 ml of dimethylsulfoxide, 5.0 g (0.023 mol) of trifluoroethyl iodide ($CH_3CH_2I$) was added to the solution. The reaction solution was heated to 50 to 70 ° C followed by stirring for 4 hours. After cooling, water was added and the mixture was extracted with 100 ml of ethyl acetate. After washing with water and drying, the extract was concentrated under reduced pressure to give about 1.2 g of crude crystals. The crystals were dissolved in n-hexane and insoluble matters were filtered off. The filtrate was then kept in a refrigerator at 3 ° C overnight. The resulting crystals were collected by filtration to give 0.6 g (yield, 17.8%) of crystals showing a melting point of 39 to 40 ° C.

Example 7

Synthesis of 3-difluoromethylthio-4-cyano-5-methylthioisothiazole (Compound 97)

After 20 ml of dioxane was added to 2.1 g (0.01 mol) of 3-mercapto-4-cyano-5-methylthioisothiazole sodium salt, 20 ml of 15% potassium hydroxide aqueous solution was added to the mixture with stirring. Difluorochloromethane was blown thereinto for 30 minutes and water was added to the system. The mixture was extracted with ethyl acetate. After washing with water and drying, the extract was concentrated under reduced pressure to give 1.5 g of crude crystals. The crystals were dissolved in n-hexane with heating and insoluble matters were filtered off. The filtrate was kept in a refrigerator at 3 ° C overnight. The resulting crystals were collected by filtration and dried to give 0.7 g (yield, 29%) of light brown crystals showing a melting point of 59 to 60 ° C.

Example 8

Synthesis of 3-difluoromethoxy-4-cyano-5-ethoxyisothiazole (Compound 122)

After 3.1 g (0.02 mol) of 3-hydroxy-4-cyano-5-ethoxyisothiazole was dissolved in 20 ml of dioxane, 40 ml of 20% sodium hydroxide aqueous solution was added to the solution and difluorochloromethane was blown thereinto for 30 minutes with stirring. After cooling, water was added to the system. The mixture was extracted with ethyl acetate. After washing with water and drying, the extract was concentrated under reduced pressure. The resulting crystals were recrystallized from ether to give 1.5 g (yield, 36%) of white crystals showing a melting point of 65 to 66 ° C.

Example 9

Synthesis of 3-trifluoroethoxy-4-cyano-5-benzyloxyisothiazole(Compound 167)

After 1.8 g (0.01 mol) of 3-hydroxy-4-cyano-5-benzyloxyisothiazole was dissolved in 20 ml of dimethylformamide, 0.7 g of potassium carbonate and 3.0 g (0.014 mol) of trifluoroethyl iodide were added to the solution. The mixture was stirred at 50 to 70 ° C for 6 hours. After cooling, water was added. The mixture was extracted with ethyl acetate. After washing with water and drying, the extract was concentrated under

reduced pressure to give viscous oily substance. The oily substance was dissolved in ether and the solution was kept in a refrigerator at 3°C overnight. The resulting crystals were collected by filtration and dried to give 0.5 g (yield, 18%) of white crystals showing a melting point of 71 to 72°C.

Example 10

Synthesis of 3-difluoromethoxy-4-cyano-5-phenoxyisothiazole (Compound 132)

After 2.2 g (0.01 mol) of 3-hydroxy-4-cyano-5-phenoxyisothiazole was dissolved in 20 ml of xylene, 40 ml of 15% potassium hydroxide aqueous solution was added to the solution. While stirring, difluorochloromethane was blown into the mixture for 30 minutes. After cooling, the xylene phase was fractionated, washed with water, dried and concentrated under reduced pressure to give viscous oily substance. The oily substance was dissolved in ether-hexane and the solution was kept in a refrigerator at 3°C overnight. The resulting crystals were collected by filtration to give 1.6 g (yield, 59%) of white crystals showing a melting point of 54 to 55°C.

Example 11

Synthesis of 3-difluoromethoxy-4-cyano-5-phenylthioisothiazole (Compound 52)

After 2.4 g (0.01 mol) of 3-hydroxy-4-cyano-5-phenylthioisothiazole was dissolved in 20 ml of dimethoxyethane, 20 ml of 30% potassium hydroxide aqueous solution was added to the solution. While stirring, difluorochloromethane was blown into the mixture for 30 minutes. After cooling, 100 ml of ethyl acetate and 200 ml of water were added to the reaction mixture followed by extraction. The ethyl acetate phase was fractionated, washed with water, dried and concentrated under reduced pressure to give viscous oily substance. The oily substance was solidified to give 1.5 g of crystals showing a melting point of 93 to 94°C.

Example 12

Synthesis of 3-difluoromethoxy-4-cyano-5-pyrimidylthioisothiazole (Compound 62)

After 2.68 g (0.01 mol) of 3-difluoromethoxy-4-cyano-5-ethylsulfonylisothiazole was dissolved in 10 ml of dimethylformamide, 1.12 g (0.01 mol) of 2-mercaptopyrmidine was added to the solution at room temperature and then 20 ml of 20% sodium hydroxide aqueous solution was dropwise added to the mixture, with stirring. After stirring for 30 minutes, 100 ml of water was added to the reaction solution. The resulting crystals were collected by filtration, washed with water and dried. The crystals were recrystallized from ethyl acetate to give 2.6 g (yield, 92%) of white crystals showing a melting point of 147°C.

Example 13

Synthesis of 3-tetrafluoroethoxy-4-cyano-5-isopropylthioisothiazole (Compound 78)

After 2.0 g (0.01 mol) of 3-hydroxy-4-cyano-5-isopropylthioisothiazole was dissolved in 20 ml of dimethylsulfoxide, 0.7 g of potassium carbonate was added to the solution. While stirring 7.2 g (0.03 mol) of tetrafluoropropyl iodide was added to the mixture. The mixture was heated to 70 to 80°C followed by stirring for 2 hours. After cooling, water was added. The mixture was extracted with ethyl acetate. After washing with water and drying, the extract was concentrated under reduced pressure to give viscous oily substance. The oily substance was solidified at room temperature to give 1.2 g of crystals showing a melting point of 39 to 40°C.

Example 14

Synthesis of 3-difluoromethylthio-4-cyano-5-sec-butylthioisothiazole (Compound 102)

After 2.5 g (0.01 mol) of 3-mercapto-4-cyano-5-sec-butylthioisothiazole sodium salt was mixed with 10 ml of dioxane and 20 ml of 30% potassium hydroxide aqueous solution. While stirring, difluorochloromethane was blown into the mixture at room temperature for 30 minutes to react them. By adding water

to the reaction mixture, the oily substance was separated out followed by extraction with ethyl acetate. The extract was washed with water, dried and concentrated under reduced pressure. The resulting oily substance was purified by silica gel column chromatography to give 1.5 g (yield, 53%) of the oily substance.
$n_D^{23}$ : 1.5641

Example 15

Synthesis of 3-sec-butylthio-4-cyano-5-difluoromethylthioisothiazole (Compound 120)

After 2.5 g (0.01 mol) of 3-sec-butylthio-4-cyano-5-mercaptoisothiazole sodium salt was mixed with 10 ml of dioxane and 20 ml of 30% potassium hydroxide aqueous solution. While stirring, difluorochloromethane was blown into the mixture at room temperature for 30 minutes to react them. By adding water to the reaction mixture, the oily substance was separated out followed by extraction with ethyl acetate. The extract was washed with water, dried and concentrated under reduced pressure. The resulting oily substance was dissolved in n-hexane followed by purification using silica gel column chromatography; 1.4 g (yield, 49%) of the oily substance was obtained.
$n_D^{23}$ : 1.5641

Example 16

Synthesis of 3-difluoromethoxy-4-cyano-5-isopropylaminoisothiazole (Compound 176)

After 1.8 g (0.01 mol) of 3-hydroxy-4-cyano-5-isopropylaminoisothiazole sodium salt was mixed with 20 ml of dioxane and 20 ml of 30% potassium hydroxide aqueous solution was added to the mixture. While stirring, difluorochloromethane was blown into the mixture at room temperature for 30 minutes to react them. After adding 100 ml of water, the mixture was extracted with ethyl acetate. The extract was washed with water, dried and concentrated under reduced pressure. The resulting crude crystals were recrystallized from ether to give 1.8 g (yield, 77%) of crystals showing a melting point of 160 to 161 °C.

Example 17

Synthesis of 3-difluoromethoxy-4-cyano-5-(3-methylphenoxy)isothiazole (Compound 135)

After 1.0 g (0.004 mol) of 3-difluoromethoxy-4-cyano-5-methylsulfonylisothiazole was dissolved in 20 ml of dimethylformamide, a mixture of 0.5 g of m-cresol and 0.93 g of 30% sodium hydroxide aqueous solution in 5 ml of dimethylformamide was dropwise added to the mixture at room temperature, with stirring. After completion of the dropwise addition, the mixture was heated to 40 to 50 °C followed by stirring for 30 minutes. After cooling, water was added to the reaction solution followed by extraction with ethyl acetate. The extract was washed with water, dried and then concentrated under reduced pressure to give a viscous substance. The substance was dissolved in ether-hexane. The solution was kept in a refrigerator at 3 °C for 2 days. The resulting crystals were collected by filtration to give 0.6 g of white crystals showing a melting point of 58 to 59 °C.

Example 18

Synthesis of 3-difluoromethoxy-4-cyano-5-(p-chlorophenylthio)isothiazole (Compound 54)

After 1.0 g (0.004 mol) of 3-difluoromethoxy-4-cyano-5-methylsulfonylisothiazole was dissolved in 30 ml of acetone, 0.6 g (0.0041 mol) of p-chlorothiophenol was added to the solution. While stirring, 0.84 g of 20% sodium hydroxide aqueous solution was dropwise added to the mixture at room temperature. After completion of the dropwise addition, the mixture was heated to 40 to 50 °C followed by stirring for an hour. After cooling, water was added to the reaction solution and the resulting crystals were collected by filtration, washed with water, and dried. The crystals were recrystallized from ether to give 0.9 g (yield, 70%) of white crystals showing a melting point of 132 to 133 °C.

Example 19

Synthesis of 3-difluoromethoxy-4-cyano-5-diethylaminodithiocarbonylisothiazole (Compound 40)

After 1.0 g (0.004 mol) of 3-difluoromethoxy-4-cyano-5-methylsulfonylisothiazole was dissolved in 10 ml of dimethylformamide, 1.0 g (0.006 mol) of sodium diethylaminocarbodithiolate was added to the solution at room temperature with stirring. The reaction mixture was heated to 40 to 50°C and stirred for an hour. Thereafter water was added and the resulting crystals were collected by filtration and dried. The crystals were recrystallized from ethyl acetate to give 0.9 g of white crystals showing a melting point of 91 to 92°C.

$^1$HNMR ($CDCl_3$) $\sigma$: 1.30-1.35 (6H, t), 3.49-3.56 (4H, q), 6.97-7.45 (1H, t, J = 7.1 Hz)

Example 20

Synthesis of 3-difluoromethoxy-4-cyano-5-acetylthioisothiazole (Compound 37)

After 0.54 g (0.002 mol) of 3-difluoromethoxy-4-cyano-5-ethylsulfonylisothiazole was dissolved in 10 ml of dimethoxyethane, 0.2 g of thioacetic acid was added to the solution and then 0.11 g of 60% sodium hydride was added thereto. After stirring for 3 hours at room temperature, water was added to the reaction solution followed by extraction with ethyl acetate. The extract was washed with water and dried and then the ether was distilled off. The residue was washed with hexane and recrystallized from ether to give 0.32 g of white crystals showing a melting point of 82 to 83°C.

Example 21

Synthesis of 3-methoxy-4-cyano-5-difluoromethoxyisothiazole (Compound 151)

After 1.3 g (0.005 mol) of 3-methoxy-4-cyano-5-ethylsulfonylisothiazole was dissolved in 10 ml of dioxane, 10 ml of 30% potassium hydroxide aqueous solution was added to the solution. While stirring, difluorochloromethane was blown into the mixture to react them. After reacting for an hour, water was added to the reaction solution followed by extraction with 50 ml of ethyl acetate. The extract was washed with water, dried and then concentrated under reduced pressure to give 0.4 g of crude crystals. The crude crystals were recrystallized from ether to give 0.25 g (yield 12%) of white crystals showing a melting point of 32 to 33°C.

$^1$HNMR ($CDCl_3$) $\sigma$: 4.05 (3H, s), 6.50-6.99 (1H, t, J = 7.5 Hz)

Example 22

Synthesis of 3-methoxy-4-cyano-5-difluoromethylthioisothiazole (Compound 86)

After 1.3 g (0.005 mol) of 3-methoxy-4-cyano-5-ethylsulfonylisothiazole was dissolved in 10 ml of dioxane, a solution of 0.6 g of potassium hydrosulfide in 5 ml of water was added to the solution at room temperature with stirring. Twenty minutes after, 10 ml of 30% potassium hydroxide aqueous solution was dropwise added to the mixture over 30 minutes with stirring, while blowing difluorochloromethane into the system. After completion of the dropwise addition, difluorochloromethane was blown into the mixture for further 10 minutes. Then water was added to the reaction solution followed by extraction with ethyl acetate. The extract was washed with water, dried and then concentrated under reduced pressure to give a brown oily substance. The oily substance was dissolved in hexane. The solution was kept at 3°C in a refrigerator overnight. The resulting crystals were collected by filtration and dried to give 0.2 g of light brown crystals showing a melting point of 29 to 30°C.

$^1$HNMR ($CDCl_3$) $\sigma$: 4.11 (3H, s), 6.80-7.17 (1H, t, J = 5.4 Hz)

Example 23

Synthesis of 3-(1,1,1-trifluoroethoxy)-4-cyano-5-acetomethylthioisothiazole (Compound 76)

After 2.96 g (0.01 mol) of 3-(1,1,1-trifluoroethoxy)-4-cyano-5-ethylsulfonylisothiazole was dissolved in 10 ml of dimethoxyethane, a solution of 2.0 g of sodium hydrosulfide in 10 ml of water was added to the solution at room temperature with stirring. After stirring for 30 minutes, calcium carbonate was added to the

mixture. Then 1.0 g of monochloroacetone was added thereto followed by stirring for an hour at 40 to 50 °C. After cooling, water was added followed by extraction with ethyl acetate. The extract was washed with water, dried and then concentrated under reduced pressure to give an oily substance. The oily substance was dissolved in hexane and insoluble matters were removed. The solution was kept at 3 °C in a refrigerator overnight. The resulting crystals were collected by filtration to give 0.2 g of white crystals showing a melting point of 51 to 52 °C.

$^1$HNMR ($CDCl_3$) σ: 2.38 (3H, s), 4.04 (2H, s), 4.61-4.80 (2H, q)

Example 24

Synthesis of 3-difluoromethoxy-4-cyano-5-propargylthioisothiazole (Compound 15)

After 2.6 g (0.01 mol) of 3-difluoromethoxy-4-cyano-5-ethylsulfonylisothiazole was dissolved in 30 ml of tetrahydrofuran, a solution of 2.0 g of sodium hydrosulfide in 10 ml of water was added to the solution. After stirring at room temperature for 30 minutes, 0.7 g of calcium carbonate was added to the mixture. Then a solution of 1.2 g of propargyl bromide in 5 ml of tetrahydrofuran was dropwise added thereto. The mixture was then heated to 40 to 50 °C followed by stirring for an hour. The reaction mixture was carefully poured onto water followed by extraction with ethyl acetate. The extract was washed with water, dried and then concentrated under reduced pressure to give an oily substance. The oily substance was dissolved in hexane and insoluble matters were filtered off. The filtrate was kept at 3 °C in a refrigerator overnight. The resulting crystals were collected by filtration to give 0.8 g of crystals showing a melting point of 39 to 41 °C.

Example 25

Synthesis of 3-difluoromethoxy-4-cyano-5-triazoloisothiazole (Compound 190)

After 2.6 g (0.01 mol) of 3-difluoromethoxy-4-cyano-5-ethylsulfonylisothiazole was dissolved in 30 ml of acetone, a solution of 0.7 g (0.01 mol) of triazole in acetone was added to the solution. Then 0.4 g of 60% sodium hydride was washed with hexane and added to the mixture. After stirring for an hour at room temperature, water was added to the reaction solution followed by extraction with ethyl acetate. The extract was washed with water and dried and then concentrated under reduced pressure to give crystals. The crystals were recrystallized from ether to give 2.2 g (yield, 85%) of white crystals showing a melting point of 56 to 57 °C.

Example 26

Synthesis of 3-difluoromethylthio-4-cyano-5-benzylthioisothiazole (Compound 105)

After 2.9 g (0.01 mol) of 3,5-bis(ethylsulfonyl)-4-cyanoisothiazole was dissolved in 20 ml of dry dimethoxyethane, 1.2 g (0.01 mol) of benzylmercaptan was added to the solution. While stirring, 0.4 g of 60% sodium hydride washed with hexane was added to the mixture. After stirring for an hour at room temperature and for 30 minutes at 50 °C, were added 2.0 g of sodium hydrosulphide, 10 ml of water thereto and stirring further for 20 minutes, then 10 ml of 30% potassium hydroxide aqueous solution was further added to the system. Then difluorochloromethane was blown into the system for 30 minutes. After cooling, water was added to the reaction solution followed by extraction with ethyl acetate. The extract was washed with water and dried and then concentrated under reduced pressure to give crystals. The resulting crude crystals were dissolved in n-hexane and insoluble matters were filtered off. The filtrate was kept at 3 °C in a refrigerator. The thus obtained crystalls were collected by filtration to give 0.2 g of crystals showing a melting point of 44 to 46 °C.

Example 27

Synthesis of 3-(1,1,1-trifluoroethoxy)-4-cyano-5-benzylaminoisothiazole (Compound 192)

After 2.9 g (0.01 mol) of 3,5-bis(ethylsulfonyl)-4-cyanoisothiazole was reacted with 1.07 g (0.01 mol) of benzylamine in 20 ml of dimethylformamide, 1.0 g (0.01 mol) of trifluoroethanol was reacted with sodium hydride and the resulting sodium trifluoroethoxide was gradually added to the reaction mixture. After reacting for 2 hours at room temperature, water was added followed by extraction with ethyl acetate. The

extract was washed with water, dried and then concentrated under reduced pressure to give crude crystals. The crude crystals were recrystallized from n-hexane to give 2.5 g (yield, 79%) of white crystals showing a melting point of 51 to 52 °C.

Example 28

Synthesis of 3,5-bis(trifluoroethoxy)-4-cyanoisothiazole (Compound 166)

After 2.9 g (0.01 mol) of 3,5-bis(ethylsulfonyl)-4-cyanoisothiazole was dissolved in 20 ml of tetrahydrofuran, 2.0 g (0.02 mol) of trifluoroethanol was reacted with sodium hydride and the resulting trifluoroethoxide was gradually added to the reaction mixture. After reacting for 2 hours at 40 to 50 °C, water was added followed by extraction with ethyl acetate. The extract was washed with water, dried and then concentrated under reduced pressure to give crystals. The crystals were recrystallized from n-hexane to give 2.5 g of crystals showing a melting point of 46 to 47 °C.

Example 29

Synthesis of 3-pentafluoropropoxy-4-cyano-5-phenoxyisothiazole (Compound 170)

After 2.9 g (0.01 mol) of 3-ethylsulfonyl-4-cyano-5-phenoxy-isothiazole was dissolved in 20 ml of dimethylformamide, 1.5 g (0.01 mol) of 2,2,3,3,3-pentafluoropropyl alcohol was added to the solution. While stirring, 0.4 g of 60% sodium hydride was added thereto after washing sodium hydride with hexane. The mixture was stirred at room temperature. The reaction mixture was poured onto water followed by extraction with ethyl acetate. The extract was washed with water, dried and then concentrated under reduced pressure to give crude crystals. The crystals were recrystallized from n-hexane to give 2.6 g of white crystals showing a melting point of 45 to 47 °C.

Hereafter synthesis examples for preparing the starting compounds of the compounds in accordance with the present invention are shown below.

Synthesis Example 1

Synthesis of 3-hydroxy-4-cyano-5-methylsulfonylisothiazole

After 1.72 g (0.01 mol) of 3-hydroxy-4-cyano-5-methylthioisothiazole was mixed with 15.4 g (0.025 mol) of oxon ($2KHSO_5 \cdot KHSO_4 \cdot K_2SO_4$), 20 ml of water was added to the mixture. With stirring 4 ml of conc. sulfuric acid was dropwise added to the mixture and then stirred for 15 hours. Water was added to the reaction solution and the resulting crystals were filtered and washed with water. The crystals were washed with ethyl acetate and recrystallized from acetone to give 1.5 g (yield, 79%) of white crystals showing a melting point of 225 °C (dec.).

Synthesis Example 2

Synthesis of 3-difluoromethoxy-4-cyano-5-methylsulfonylisothiazole

After 5 ml of ethanol, 15.4 g (0.025 mol) of oxon and 10 ml of water were added to 2.2 g (0.01 mol) of 3-difluoromethoxy-4-cyano-5-methylthioisothiazole. With stirring 4 ml of conc. sulfuric acid was dropwise added to the mixture. After the dropwise addition, the reaction solution was solidified and hence, water was additionally dropwise added to the reaction solution until stirring could be performed. Heat generated so that the temperature of the reaction solution became 50 to 60 °C, but the reaction solution was vigorously stirred for 6 hours as it was. The reaction solution was poured onto water and the crystals were filtered and washed with water. The crystals were recrystallized from ethyl acetate to give 2.0 g (yield, 78%) of white crystals showing a melting point of 108 to 109 °C.

Synthesis Example 3

Synthesis of 3-difluoromethoxy-4-cyano-5-ethylsulfonylisothiazole

To 2.0 g (0.05 mol) of 3-difluoromethoxy-4-cyano-5-ethylthioisothiazole was added 30 ml of acetic acid. Furthermore 70 g (0.11 mol) of oxon and 40 ml of water were added to the mixture followed by vigorous stirring. The reaction solution showed 50 to 60°C, but the reaction solution was vigorously stirred for 12 hours as it was. After cooling, water was added thereto. The resulting crystals were filtered, washed with water and dried to give 12.2 g of crystals. The crystals were recrystallized from ether to give 2.0 g (yield, 78%) of white crystals showing a melting point of 52 to 53°C.

Synthesis Example 4

Synthesis of 3-(1,1,1-trifluoroethoxy)-4-cyano-5-ethylsulfonylisothiazole

3-(1,1,1-Trifluoroethoxy)-4-cyano-5-ethylthioisothiazole was oxidized with oxon in a manner similar to Synthesis Example 3. Recrystallization from n-hexane gave white crystals showing a melting point of 40 to 41°C.

Synthesis Example 5

Synthesis of 3-hydroxy-4-cyano-5-benzyloxyisothiazole

After 4.1 g (0.02 mol) of 3-hydroxy-4-cyano-5-methylsulfonylisothiazole was dissolved in 20 ml of tetrahydrofuran, 2.5 g (0.023 mol) of benzyl alcohol and 9.2 g of 30% potassium hydroxide aqueous solution were added to the solution at room temperature with stirring. While stirring, the temperature was elevated to 40 to 50°C, where the reaction was carried out for 2 hours. After cooling, water was added and 40 ml of 10% hydrochloric acid was further added to render the system acidic followed by concentration under reduced pressure. The thus obtained residue was extracted twice with 100 ml of ethyl acetate. The extract was dried and concentrated under reduced pressure. Ether was added to the residue to precipitate crystals. The crystals were filtered to give 2.8 g (yield, 56%) of white crystals having a melting point of 160 to 162°C.

Synthesis Example 6

Synthesis of 3-hydroxy-4-cyano-5-dimethylaminoisothiazole

After 4.1 g (0.02 mol) of 3-hydroxy-4-cyano-5-methylsulfonylisothiazole was dissolved in 30 ml of tetrahydrofuran, 1.0 g of dimethylamine was dropwise added to the solution at room temperature with stirring. After the dropwise addition, the mixture was heated to 40 to 50°C and reacted for 2 hours. The reaction solution was concentrated under reduced pressure. The resulting crude powdery residue was extracted with acetone and the acetone was concentrated under reduced pressure. The resulting crystals were washed with ether to give 1.1 g (yield, 56%) of white crystals having a melting point of 260°C (dec.).

Synthesis Example 7

Synthesis of 3-hydroxy-4-cyano-5-phenylthioisothiazole

After 2.0 g (0.01 mol) of 3-hydroxy-4-cyano-5-methylsulfonylisothiazole was dissolved in 20 ml of dimethylformamide, a mixture of 1.2 g of thiophenol and 4.0 g of 30% potassium hydroxide aqueous solution in 5 ml of dimethylformamide was dropwise added to the solution. After stirring for 2 hours at about 50°C, water was added and 20 ml of 10% hydrochloric acid was then added to render the system acidic. The resulting crystals were filtered, washed with water and dried. The crystals were recrystallized from ether to give 1.5 g (yield, 63%) of white crystals having a melting point of 215 to 220°C.

Compounds shown in Table 2 were synthesized as in Synthesis Examples 5 through 7.

Table 2

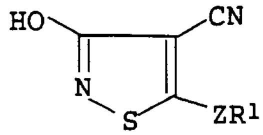

| Synthesis Example No. | $ZR^1$ | Physical Property m.p. (°C) |
|---|---|---|
| 8 | 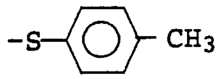 | 196-197 |
| 9 | 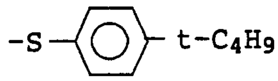 | 146-147 |
| 10 | 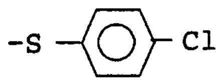 | 231-233 |
| 11 | 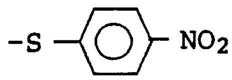 | 255 (dec.) |
| 12 | 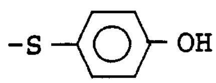 | 182-183 |
| 13 | 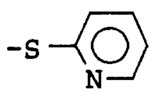 | 230 (dec.) |
| 14 | 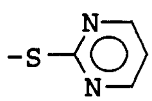 | 250 (dec.) |
| 15 | 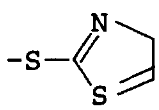 | 189-191 |
| 16 | 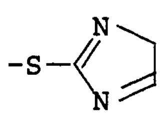 | ~220 (dec.) |
| 17 | 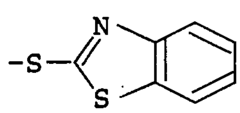 | ~240 (dec.) |

Table 2 (Cont'd)

| Synthesis Example No. | $ZR^1$ | Physical Property m.p. (°C) |
|---|---|---|
| 18 | 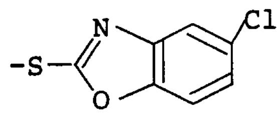 | ~250 (dec.) |
| 19 | 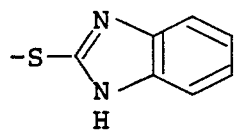 | ~250 (dec.) |
| 20 | 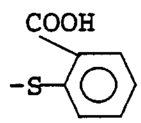 | 215 (dec.) |
| 21 | 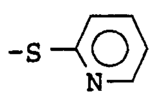 | 200 (dec.) |
| 22 | 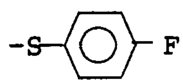 | 211-213 |
| 23 | 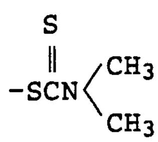 | 260 (dec.) |
| 24 | 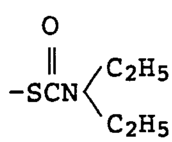 | 230 (dec.) |
| 25 | 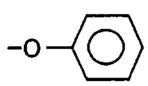 | 152 |
| 26 | 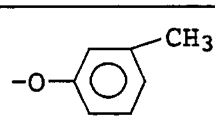 | 113-114 |
| 27 | 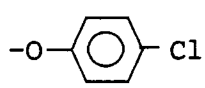 | 168-169 |

Table 2 (Cont'd)

| Synthesis Example No. | $ZR^1$ | Physical Property m.p. (°C) |
|---|---|---|
| 28 | 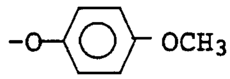 | 170-171 |
| 29 | 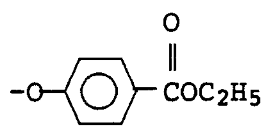 | 109-110 |
| 30 | 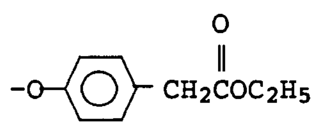 | 98-99 |
| 31 | 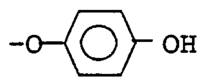 | ~150 (dec.) |
| 32 | 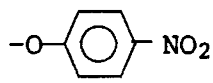 | 230 (dec.) |
| 33 | 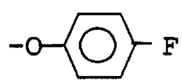 | 111-112 |
| 34 | 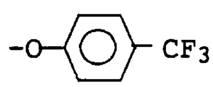 | 136-137 |
| 35 | 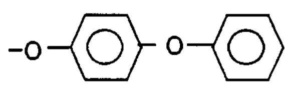 | 142-143 |
| 36 | 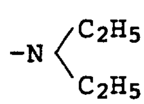 | 220 (dec.) |
| 37 | 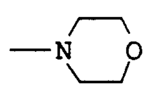 | 161-163 |
| 38 | 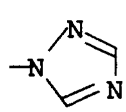 | 240 (dec.) |

In practice of the present invention, for efficiently exhibiting the effect of controlling termites, the compounds represented by general formula (I) are prepared into the form of oil spray, an emulsifiable concentrate, a solution, granules, wettable powders or a flowable concentrate, etc., in a conventional manner. As occasion demands, solid carriers or liquid carriers are used for preparing these preparations. As the adaptable solid carriers, may be cited clays (e.g., kaolin, bentonite, and acid clay), talcs (e.g., talc and

pyrophillite), siliceous substances (e.g., diatomaceous earth, silica sand, mica, synthetic silicates, highly dispersed synthetic silicic acid), inorganic mineral powders such as pumice, sand, etc. As the adaptable liquid carrier, there are the following carriers which may be used alone or as an admixture of two or more in combination: alcohols (e.g., methyl alcohol, ethyl alcohol, ethyleneglycol), ketones (e.g., acetone, methyl ethyl ketone, cyclohexanone), ethers (e.g., ethyl ether, dioxane, tetrahydrofuran, cellosolve), aliphatic hydrocarbons (e.g., gasoline, kerosine), aromatic hydrocarbons (e.g., benzene, toluene, xylene, solvent naphtha, cyclohexanone, methylnaphthalene), halogenated hydrocarbons (e.g., dichloroethane, chloroform, carbon tetrachloride, chlorobenzene), etc.

As surface active agents which can be used for the controlling composition of the present invention, the following surfactants may be mentioned but the present invention is not deemed to be limited thereto. These surfactants may be used alone or as an admixture of two or more: polyoxyethylene alkylaryl ethers, polyoxyethylene sorbitan monolaurates, alkylaryl sorbitan monolaurates, alkyl benzenesulfonates, alkyl naphthalenesulfonates, lignin sulfonates, higher alcohol sulfate esters, etc.

As dispersing agents or binders which can be used in the controlling composition of the present invention, the following substances may be used but the present invention is not deemed to be limited thereto: casein, gelatin, starch, alginic acid, CMC, gum arabic, agar, polyvinyl alcohol, turpentine oil, rice bran oil, bentonite, lignin, sulfite waste, etc.

The composition of the present invention for controlling termites is applied to the soil or the place where termites live. For protecting wooden materials themselves such as living trees, fences, stakes, railroad ties, etc., buildings such as shrines and temples, houses, barns, factories, the controlling composition may be applied not only to these wooden materials per se but around these buildings, the surface of soil under the floor, or in the soil around the buildings or under the floor thereof. The controlling composition may also be applied to wooden products such as plywood, lumbering products, particle boards, half boards, etc., polyvinyl products such as coated wires, sheets, etc. The present invention also covers such an embodiment of preliminarily treating the sites, as being preventing, that are expected to cause birth of termites.

The termite controlling composition of the present invention may be applied directly to the surface of soil or by diluting the composition with water, etc. The soil is plowed up on the surface and thoroughly blended with each other, grooves are formed around the affected site and the controlling composition is applied to the inside of the grooves. If necessary and desired, the controlling composition may also be blended into the embedded soil. For such application, there may be used the composition containing a dose of 3 g to 60 g per $m^2$ as an effective component, in the case of soil treatment.

In the case that wooden portions or the like are directly treated, the controlling composition may be applied directly or, may be applied by spraying, coating, immersion, etc. after diluting the controlling composition with water or the like. It is sufficient to use the controlling composition containing 2 g to 40 g per $m^2$ of the wooden portion.

The termite controlling composition of the present invention may be applied together with commercially available foaming agents thereby to perform foaming treatment.

In the case that the termite controlling composition of the present invention comprising as an effective ingredient the compounds represented by general formula (I) is used, it is also possible to apply the composition in combination with other termite controlling agents or wood preservatives, for the purposes of reducing the dosage or increasing the effect, of the effective ingredient. Examples of other termite controlling agents are organic phosphorous agents such as chlorpyrifos, pyridafenthion, phoxim; pyrethroidal agents such as permethrin, fenvalerate, fenpropathrin; tripropyl isocyanulate, and carbamate agents; examples of the wood preservatives include 3-iodo-2-propynylbutyl carbamate, 3-iodopropargyl, zinc naphthenate, etc.

The following examples illustrate test examples and formulation examples, but the present invention is not deemed to be limited to these examples.

FORMULATION EXAMPLE 1

Granules are obtained by uniformly mixing and dissolving the following components, and spraying the solution onto 85 parts of pumice granules.

| | |
|---|---|
| Compound of this invention | 8 parts |
| Cyclohexanone | 4 parts |
| Mixture of polyoxyethylene nonylphenyl ether and alkylbenzenesulfonic acid | 3 parts |

FORMULATION EXAMPLE 2

Oil spray is obtained by uniformly mixing and dissolving the following components.

| | |
|---|---|
| Compound of this invention | 0.5 part |
| Xylene | 0.8 part |
| Illuminating kerosine | 98.7 parts |

FORMULATION EXAMPLE 3

An emulsifiable concentrate is obtained by uniformly mixing and dissolving the following components.

| | |
|---|---|
| Compound of this invention | 25 parts |
| Xylene | 60 parts |
| Mixture of polyoxyethylene nonylphenyl ether and alkylbenzenesulfonic acid | 15 parts |

FORMULATION EXAMPLE 4

Wettable powders are obtained by uniformly mixing and grinding the following components.

| | |
|---|---|
| Compound of this invention | 50 parts |
| Mixture of diatomaceous earth and clay | 45 parts |
| Polyoxyethylene nonylphenyl ether | 5 parts |

FORMULATION EXAMPLE 5

Dust is obtained by uniformly mixing and dissolving the following components.

| | |
|---|---|
| Compound of this invention | 4 parts |
| Mixture of diatomaceous earth, clay and talc | 95 parts |
| Calcium stearate | 1 part |

Next, the effect of controlling termites in the present invention is explained with reference to test examples. However, the present invention is not deemed to be limited thereto.

TEST EXAMPLE 1

A filter paper is put on a Petri dish having a diameter of 9 cm and 1.5 ml of the composition diluted to a given concentration is carefully poured thereon. 20 Formosan subterranean termites (Coptotermes formosanus) are put in the Petri dish. The dish is kept in a thermostat at 28°C and a rate of dead termites is determined 7 days after: 20 termites in one group and 3 series of test. The results are shown in Table 3.

Rate of dead termites

100% : A
99-70% : B
69-50% : C
less than 49% : D

Table 3

| Compound No. | Concentration of chemical | | Compound No. | Concentration of chemical | |
|---|---|---|---|---|---|
| | 0.05% | 0.01% | | 0.05% | 0.01% |
| 1 | A | A | 86 | A | A |
| 2 | A | A | 88 | A | A |
| 4 | A | A | 95 | A | A |
| 7 | A | A | 97 | A | A |
| 8 | A | A | 100 | A | A |
| 9 | A | A | 102 | A | A |
| 10 | A | A | 103 | A | A |
| 11 | A | A | 108 | A | B |
| 17 | A | A | 113 | A | B |
| 18 | A | A | 117 | A | A |
| 19 | A | B | 121 | A | B |
| 22 | A | C | 124 | A | C |
| 25 | A | B | 128 | A | B |
| 28 | A | B | 132 | A | B |
| 32 | A | A | 135 | A | A |
| 36 | A | A | 137 | A | A |
| 40 | A | A | 144 | A | B |
| 47 | A | A | 146 | A | B |
| 49 | A | A | 147 | A | A |
| 52 | A | A | 152 | A | A |
| 55 | A | A | 154 | A | A |
| 61 | A | B | 156 | A | A |
| 63 | A | B | 160 | A | B |
| 66 | A | B | 163 | A | A |
| 69 | A | C | 168 | A | A |
| 71 | A | A | 174 | A | B |
| 73 | A | A | 178 | A | B |
| 75 | A | A | 181 | A | B |
| 77 | A | B | 188 | A | B |

TEST EXAMPLE 2

Onto 20 g of air-dried soil (collected in Kawachi Nagano-shi, Japan), 4 ml of the composition diluted to a given concentration is carefully poured. After thoroughly mixing, the soil is charged in a brown bottle of 50 ml volume. The bottle is covered with an aluminum foil and kept in a thermostat room of 25°C under humidity of 60 to 70%. One month after the treatment, 10 g of the treated soil is taken and spread onto a Petri dish and 10 Formosan subterranean termites (Coptotermes formosanus) are put in the dish. After the dish is kept in a thermostat room of 28°C under humidity of 60 to 70%, a rate of dead termites is examined 7 days after. The rate of dead termites is expressed as in TEST EXAMPLE 1. The results are shown in Table 4.

Table 4

| Compound No. | Concentration of chemical 0.05% | Compound No. | Concentration of chemical 0.05% | Compound No. | Concentration of chemical 0.01% |
|---|---|---|---|---|---|
| 1 | A | 55 | A | 121 | B |
| 2 | A | 61 | C | 124 | A |
| 3 | A | 63 | B | 128 | A |
| 7 | A | 66 | B | 132 | A |
| 8 | A | 69 | B | 135 | A |
| 9 | A | 71 | A | 137 | A |
| 10 | A | 73 | A | 144 | B |
| 11 | A | 75 | A | 146 | B |
| 17 | A | 77 | B | 147 | B |
| 18 | A | 81 | B | 152 | B |
| 19 | B | 86 | B | 154 | B |
| 22 | C | 88 | B | 156 | B |
| 25 | C | 95 | A | 160 | B |
| 28 | B | 97 | A | 163 | A |
| 32 | B | 100 | A | 169 | B |
| 36 | A | 102 | A | 176 | B |
| 40 | A | 103 | A | 180 | B |
| 47 | A | 108 | B | 183 | B |
| 49 | A | 113 | B | 190 | B |
| 52 | A | 117 | A | 195 | B |

As explained hereinabove, the isothiazole derivatives of the present invention are useful as agents for controlling termites.

## Claims

**1.** An isothiazole derivative represented by general formula (I):

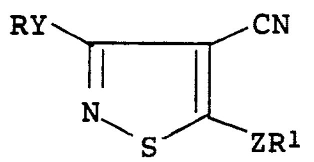

(I)

wherein either R or $R^1$ represents a fluoroalkyl group and the other represents an alkyl group having 1 to 6 carbon atoms; an alkyl group substituted with a member selected from a halogen atom, an alkoxy group having 1 to 6 carbon atoms, an alkoxycarbonyl group having 2 to 7 carbon atoms, an alkylcarbonyl group having 2 to 7 carbon atoms, benzoyl group, an alkylthio group having 1 to 6 carbon atoms, a trialkylsilyl group, a dialkylcarbamoyl group, hydroxy group, cyano group, phenylsulfonyl group, a dialkylamino group, morpholino group, an aryl group, phenoxy group and an aromatic heterocyclic group; a cycloalkyl group; an alkenyl group having 2 to 6 carbon atoms; a haloalkenyl group; an alkynyl group having 2 to 6 carbon atoms; an alkylcarbonyl group having 2 to 7 carbon atoms; benzoyl group; dialkylmethylideneimino group; a dialkylthiocarbamoyl group; a dialkylcarbamoyl group; an alkoxythiocarbonyl group having 2 to 7 carbon atoms; an aryl group or a heterocyclic group; Y represents -O- or -S-; and Z represents -O-, -S-, -N($R^2$) (wherein $R^2$ represents hydrogen atom, an alkyl group having 1 to 6 carbon atoms or an alkynyl group having 2 to 6 carbon atoms, or $R^1$ and $R^2$ may be combined together to form an alkenylene group having 2 to 6 carbon atoms which may be intervened by oxygen atom) or a single bond.

**2.** An isothiazole derivative according to claim 1, wherein R or $R^1$ represents a fluoroalkyl group and the other represents an alkyl group having 1 to 6 carbon atoms; an alkyl group substituted with a member selected from a halogen atom, an alkoxy group having 1 to 6 carbon atoms, an alkylcarbonyl group, a

trialkylsilyl group, an aryl group, phenoxy group and an aromatic heterocyclic group; a cycloalkyl group; a haloalkenyl group; an alkynyl group having 2 to 6 carbon atoms, a dialkylthiocarbamoyl group, an aryl group or a heterocyclic group, Y and Z represent -O- or -S-.

**3.** An isothiazole derivative according to claim 1 or 2, wherein R represents a fluoroalkyl group, $R^1$ represents a haloalkyl group, a trialkylsilylalkyl group, an alkoxyalkyl group, a cycloalkyl group, an alkenyl group, an alkynyl group, Y and Z represent -O-or -S-.

**4.** An isothiazole derivative according to claim 1 or 2, wherein R represents a haloalkyl group, a trialkylsilylalkyl group, an alkoxyalkyl group, a cycloalkyl group, an alkenyl group, an alkynyl group, phenyl group, an alkylphenyl group, halophenyl group, $R^1$ represents a fluoroalkyl group, Y and Z represent -O-or -S-.

**5.** A process for preparing an isothiazole derivative represented by general formula (I):

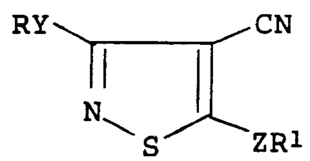

(I)

wherein either R or $R^1$ represents a fluoroalkyl group and the other represents an alkyl group having 1 to 6 carbon atoms; an alkyl group substituted with a member selected from a halogen atom, an alkoxy group having 1 to 6 carbon atoms, an alkoxycarbonyl group having 2 to 7 carbon atoms, an alkylcarbonyl group having 2 to 7 carbon atoms, benzoyl group, an alkylthio group having 1 to 6 carbon atoms, a trialkylsilyl group, a dialkylcarbamoyl group, hydroxy group, cyano group, phenylsulfonyl group, a dialkylamino group, morpholino group, an aryl group, phenoxy group and an aromatic heterocyclic group; a cycloalkyl group; an alkenyl group having 2 to 6 carbon atoms; a haloalkenyl group; an alkynyl group having 2 to 6 carbon atoms; an alkylcarbonyl group having 2 to 7 carbon atoms; benzoyl group; dialkylmethylideneimino grop; a dialkylthiocarbamoyl group; a dialkylcarbamoyl group; an alkoxythiocarbonyl group having 2 to 7 carbon atoms; an aryl group or a heterocyclic group; Y represents -O- or -S-; and Z represents -O-, -S-, -N($R^2$) (wherein $R^2$ represents hydrogen atom, an alkyl group having 1 to 6 carbon atoms or an alkynyl group having 2 to 6 carbon atoms, or $R^1$ and $R^2$ may be combined together to form an alkenylene group having 2 to 6 carbon atoms which may be intervened by oxygen atom) or a single bond; which comprises reacting a compound represented by general formula (II):

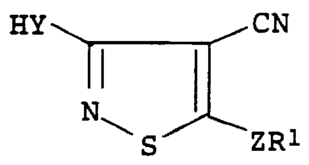

(II)

wherein $R^1$, Y and Z have the same significances as described above, with a compound represented by general formula (III):

RX (III)

wherein R has the same significance as described above and X represents a halogen atom, in the presence of a base.

**6.** A process for preparing an isothiazole derivative represented by general formula (Ia):

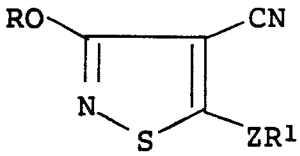

(Ia)

wherein either R or $R^1$ represents a fluoroalkyl group and the other represents an alkyl group having 1 to 6 carbon atoms; an alkyl group substituted with a member selected from a halogen atom, an alkoxy group having 1 to 6 carbon atoms, an alkoxycarbonyl group having 2 to 7 carbon atoms, a alkylcarbonyl group having 2 to 7 carbon atoms, benzoyl group, an alkylthio group having 1 to 6 carbon atoms, a trialkylsilyl group, a dialkylcarbamoyl group, hydroxy group, cyano group, phenylsulfonyl group, a dialkylamino group, morpholino group, an aryl group, phenoxy group and an aromatic heterocyclic group; a cycloalkyl group; an alkenyl group having 2 to 6 carbon atoms; a haloalkenyl group; an alkynyl group having 2 to 6 carbon atoms; an alkylcarbonyl group having 2 to 7 carbon atoms; benzoyl group; a dialkylthiocarbamoyl group; a dialkylcarbamoyl group; an alkoxythiocarbonyl group having 2 to 7 carbon atoms; an aryl group or a heterocyclic group; and Z represents - O-, -S-, -N($R^2$) (wherein $R^2$ represents hydrogen atom, an alkyl group having 1 to 6 carbon atoms or an alkynyl group having 2 to 6 carbon atoms, or $R^1$ and $R^2$ may be combined together to form an alkenylene group having 2 to 6 carbon atoms which may be intervened by oxygen atom) or a single bond, which comprises reacting a compound represented by general formula (IV):

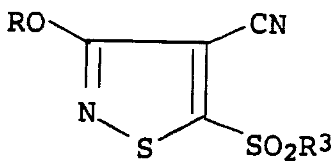

(IV)

wherein R has the same significance as described above, and $R^3$ represents an alkyl group having 1 to 6 carbon atoms; with a compound represented by general formula (V):

$R^1ZH$ (V)

wherein $R^1$ and Z have the same significances as described above; in the presence of a base.

**7.** A process for preparing an isothiazole derivative represented by general formula (Ib):

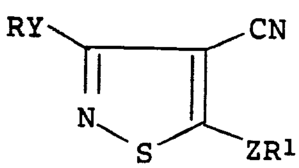

(Ib)

wherein either R or $R^1$ represents a fluoroalkyl group on the other represents an alkyl group having 1 to 6 carbon atoms; an alkyl group substituted with a member selected from a halogen atom, an alkoxy group having 1 to 6 carbon atoms, an alkoxycarbonyl group having 2 to 7 carbon atoms, an alkylcarbonyl group having 2 to 7 carbon atoms, benzoyl group, an alkylthio group having 1 to 6 carbon atoms, a trialkylsilyl group, a dialkylcarbamoyl group, hydroxy group, cyano group, phenylsulfonyl group, a dialkylamino group, morpholino group, an aryl group, phenoxy group and an aromatic heterocyclic group; a cycloalkyl group; an alkenyl group having 2 to 6 carbon atoms; a haloalkenyl group; an alkynyl group having 2 to 6 carbon atoms; an alkylcarbonyl group having 2 to 7 carbon atoms; benzoyl group; a dialkylthiocarbamoyl group; a dialkylcarbamoyl group; an alkoxythiocarbonyl group having 2 to 7 carbon atoms; an aryl group or a heterocyclic group; Y represents -O- or -S-; and Z represents -O-, -S-, -N($R^2$) (wherein $R^2$ represents hydrogen atom, an alkyl group having 1 to 6 carbon atoms or an alkynyl group having 2 to 6 carbon atoms, or $R^1$ and $R^2$ may be combined together to form

an alkenylene group having 2 to 6 carbon atoms which may be intervened by oxygen atom) or a single bond; which comprises reacting a compound represented by general formula (VII):

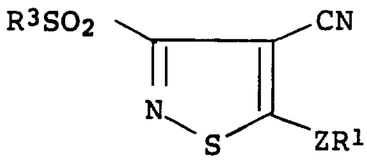

(VII)

wherein $R^1$ and Z have the same significances as described above, and $R^3$ represents an alkyl group having 1 to 3 carbon atoms; with a compound represented by general formula (VIII):

RYH (VIII)

wherein R and Y have the same significances as described above; in the presence of a base.

**8.** A composition for controlling termite comprising as an active ingredient an effective dose of an isothiazole derivative represented by general formula (I):

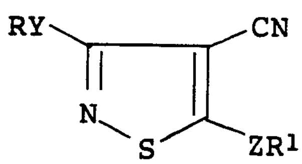

(I)

wherein either R or $R^1$ represents a fluoroalkyl group and the other represents an alkyl group having 1 to 6 carbon atoms; an alkyl group substituted with a member selected from a halogen atom, an alkoxy group having 1 to 6 carbon atoms, an alkoxycarbonyl group having 2 to 7 carbon atoms, an alkylcarbonyl group having 2 to 7 carbon atoms, benzoyl group, an alkylthio group having 1 to 6 carbon atoms, a trialkylsilyl group, a dialkylcarbamoyl group, hydroxy group, cyano group, phenylsulfonyl group, a dialkylamino group, morpholino group, an aryl group, phenoxy group and an aromatic heterocyclic group; a cycloalkyl group; an alkenyl group having 2 to 6 carbon atoms; a haloalkenyl group; an alkynyl group having 2 to 6 carbon atoms; an alkylcarbonyl group having 2 to 7 carbon atoms; benzoyl group; dialkylmethylideneimino group; a dialkylthiocarbamoyl group; a dialkylcarbamoyl group; an alkoxythiocarbonyl group having 2 to 7 carbon atoms; an aryl group or a heterocyclic group; Y represents -O- or -S-; and Z represents -O-, -S-, -N($R^2$) (wherein $R^2$ represents hydrogen atom, an alkyl group or an alkynyl group, or $R^1$ and $R^2$ may be combined together to form an alkenylene group having 2 to 6 carbon atoms which may be intervened by oxygen atom) or a single bond; and an inert carrier(s).

**9.** A composition for controlling termite according to claim 8, wherein R or $R^1$ represents a fluoroalkyl group and the other represents an alkyl group having 1 to 6 carbon atoms; an alkyl group substituted with a member selected from a halogen atom, an alkoxy group having 1 to 6 carbon atoms, an alkylcarbonyl group, a trialkylsilyl group, an aryl group, phenoxy group and an aromatic heterocyclic group; a cycloalkyl group; a haloalkenyl group; an alkynyl group having 2 to 6 carbon atoms, a dialkylthiocarbamoyl group, an aryl group or a heterocyclic group, Y and Z represents -O- or -S-.

**10.** A composition for controlling termite according to claim 8 or 9, wherein R represents a fluoroalkyl group, $R^1$ represents a haloalkyl group, a trialkylsilylalkyl group, an alkoxyalkyl group, a cycloalkyl group, an alkenyl group, an alkynyl group, Y and Z represent -O- or -S-.

**11.** A composition for controlling termite according to claim 8 or 9, wherein R represents a haloalkyl group, a trialkylsilylalkyl group, an alkoxyalkyl group, a cycloalkyl group, an alkenyl group, an alkynyl group, phenyl group, an alkylphenyl group, halophenyl group $R^1$ represents a fluoroalkyl group, Y and Z represent -O-or -S-.

European Patent Office

# EUROPEAN SEARCH REPORT

Application Number

EP 93 11 0952

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | DE-A-1 954 179 (MERCK ANLAGEN-GMBH)<br>* page 5; claim 1 *<br>--- | 1,8-11 | C07D275/03<br>A01N43/80<br>C07F7/08<br>C07D417/12 |
| A | CH-A-507 281 (MERCK-ANLAGEN-GMBH)<br>* column 2, line 33 - column 4, line 20; claims *<br>--- | 1,8-11 | |
| A | EP-A-0 174 086 (ROHM AND HAAS COMPANY)<br>*pages 3-6,example1,tables 1 and 2*<br>* claims 1,8 *<br>--- | 1,8-11 | |
| A | PATENT ABSTRACTS OF JAPAN<br>vol. 10, no. 71 (C-334)(2128) 20 March 1986<br>& JP-A-60 208 972 ( NIPPON KAYAKU K.K. ) 21 October 1985<br>* abstract *<br>----- | 1,8-11 | |

TECHNICAL FIELDS SEARCHED (Int. Cl.5)

C07D

The present search report has been drawn up for all claims

| Place of search | Date of completion of the search | Examiner |
|---|---|---|
| THE HAGUE | 29 SEPTEMBER 1993 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document